# EUROPEAN PATENT APPLICATION

(11) **EP 3 543 330 A1**
(43) Date of publication of application: **25.09.2019**
(21) Application number: 18162761.3
(22) Date of filing: 20.03.2018
(51) Int. Cl.: C12N 5/00

(54) **A METHOD FOR IMPROVING PRODUCT TITER IN A CELL AND A CELL MEDIA FOR IMPROVING THE SAME**

(71) Applicant: Valitacell Limited, Dublin (IE)
(72) Inventor: KALSI, Devika, Tralee, Co. Kerry Leebrook (IE); JAMES, David, Sheffield, South Yorkshire S119LN (GB); THOMPSON, Ben, Sheffield, South Yorkshire S3 7BG (GB); CLIFFORD, Jerry, Tralee, Co. Kerry Leebrook (IE)
(74) Representative: Purdylucey Intellectual Property

(57) **Abstract**

A cell culture media comprising an effective amount of 3-Thiopheneacetic acid (3TAA).

P12550EP00 Sequence Listing_ST25
SEQUENCE LISTING

<110>  Valitacell Limited

<120>  A method for improving product titer in a cell and a cell media
       for improving the same

<130>  P12550EP00

<160>  8

<170>  PatentIn version 3.5

<210>  1
<211>  20
<212>  DNA
<213>  Artificial Sequence

<220>
<223>  Forward Primer

<400>  1
accaagaacc aggtcagcct                                                   20


<210>  2
<211>  20
<212>  DNA
<213>  Artificial Sequence

<220>
<223>  Reverse Primer

<400>  2
tgagaagacg ttcccctgct                                                   20


<210>  3
<211>  20
<212>  DNA
<213>  Artificial Sequence

<220>
<223>  Forward Primer

<400>  3
cagcaaggac agcacctaca                                                   20


<210>  4
<211>  20
<212>  DNA
<213>  Artificial Sequence

<220>
<223>  Reverse Primer

<400>  4
gacttcgcag gcgtagactt                                                   20


<210>  5
<211>  20
<212>  DNA
<213>  Artificial Sequence

<220>
<223>  Forward Primer

[?]

<400>  5
tgtcacctca atgggactgc                                                    20


<210>  6
<211>  23
<212>  DNA
<213>  Artificial Sequence

<220>
<223>  Reverse Primer

<400>  6
caggtgcatc actactctga aac                                                23


<210>  7
<211>  20
<212>  DNA
<213>  Artificial Sequence

<220>
<223>  Forward Primer

<400>  7
ctctcgggac agaaacaagc                                                    20


<210>  8
<211>  22
<212>  DNA
<213>  Artificial Sequence

<220>
<223>  Reverse Primer

<400>  8
gacctacact catctgggct ac                                                 22

## Description

### Field of the Invention

This invention relates to a method of increasing the product titer of a producer cell. In particular, the invention relates to cell culture media comprising an effective amount of an additive for increasing the product titer of a producer cell.

### Background to the Invention

Bottlenecks in biopharmaceutical production often relate to low volumetric titers. This is normally due to insufficient cellular machinery and/or complex production proteins. Improving recombinant protein production in mammalian cells thus remains an important target for the biopharmaceutical industry. Small molecule chemical enhancer molecules present a facile avenue to quicken production boosts by relieving cellular/protein bottlenecks (see, for example, WO 2004/096833). With the rise in demand for "difficult to express proteins" and fusion antibodies, improving the cell culture process is vital.

3-Thiopheneacetic acid (3TAA) is a carboxylic acid used in the production of gold nanoparticles. Research has been conducted using 3TAA in the formation of a heterostructure of conjugated polymer and semiconducting nanoparticles and in nanostructured films. The majority of available prior art focuses on the use of 3TAA as a polymer for biosensors. 3TAA has been used as a conducting copolymer for coating on medical biosensors. Previous studies have primarily examined the usage of 3TAA polymerisation in biosensors where addition of 3TAA is used to link biological elements to sensors.

However, some small chemical enhancers are known to induce apoptosis at their optimum concentration in mammalian cell hosts and are unsuitable for enhancing the titer in a cell culture process.

It is an objective of the present invention to overcome at least one of the above-referenced problems.

### Summary of the Invention

The inventors have identified the use of 3TAA as a novel culture media additive to boost protein production (titer) in mammalian cell hosts. The inventors demonstrate the efficacy of a novel carboxylic acid, 3TAA, as an additive to improve biotherapeutic product titer in a batch production process. This invention presents a titer improvement tool that is not harmful to cell health.

According to the invention, there is provided, as set out in the appended claims, a cell culture media comprising an effective amount of 3-Thiopheneacetic acid (3TAA).

In one aspect, there is provided a producer cell culture media, comprising an effective amount of 3-Thiopheneacetic acid (3TAA).

Preferably, the effective amount of 3TAA is between about 0.1 mM and about 6.0 mM.

Preferably, the effective amount of 3TAA is between about 1.5 mM and about 3.5 mM when the cell culture media is added to cells at Day 0. Ideally, the effective amount of 3TAA is about 2.5 mM when the cell culture media added to the cells at Day 0.

Preferably, the effective amount of 3TAA is between about 4.0 mM and about 6.0 mM when the cell culture media supplement is added to the cells at when the producer cell culture media is added to the cells at mid to late exponential phase or early stationary phase of the cell growth. Ideally, the effective amount of 3TAA is about 5.6 mM when the cell culture media is added to the cells at mid to late exponential phase or early stationary phase of the cell growth.

Preferably, the producer cell culture media is where an effective amount of 3TAA is added to a basal cell culture media selected from Dulbecco's Modified Eagle Medium, Ham's F-10, Ham's F-12, Minimum Essential Medium, RPMI 1640 Media, CD CHO Media, FreeStyle™ 293 Expression Medium, CD FortiCHO medium, CD OptiCHO Medium, EX-CELL®, HyClone, Cellvento, BalanCD™ CHO Growth A, PowerCHO, ClonaCell, IS CHO-CD XP, CHOMACS CD.

Preferably, the cell culture media is protein-free and serum-free.

Preferably, the cell culture media is in the form of a powder, a liquid, a pellet, a granule.

The harvested protein may be expressed stably by the producer cell; where the cells constantly produce the protein of interest, or transiently where the cells are transfected with the gene of interest for the production of a protein of interest.

In one aspect, there is provided a method for increasing product titer in a cell culture system, the method comprising the steps of:
(a) incubating a cell in the producer cell culture media of Claim 1 for a period of time in the cell culture system;
(b) collecting the cell or cell culture media, or both, from the cell culture system; and
(c) harvesting the product produced by the producer cell in the cell culture system.

In one aspect, there is provided a method for increasing protein production of a protein-producing cell in a cell culture system, the method comprising the steps of:
(a) incubating the protein-producing cell in the producer cell culture media as described above for a period of time;
(b) collecting the producer cells or the producer cell culture media, or both, from the system; and
(c) harvesting the recombinant protein from the producer cells or the producer cell culture media, or both.

Preferably, the harvested protein is a monoclonal antibody, a polyclonal antibody or a recombinant protein.

Preferably, the method further comprises the step of incubating the cells in a basal cell culture media prior to step (a) for a period of time until the cells are at mid to late exponential phase or early stationary phase of the cell growth. When the cells are at mid to late exponential phase or early stationary phase of the cell growth, the basal cell culture media is supplemented with the producer cell culture media of Claim 1.

Preferably, the method comprises the step of incubating the cells with a first effective amount of 3TAA of at least about 0.8 mM at Day 0 and increasing the effective amount of 3TAA to about 5.6 mM at mid to late exponential phase or early stationary phase of the cell growth.

In one aspect, there is provided a method of determining whether the cell culture media described above increases product titer in a cell culture system when compared to product titer when using the cell culture media without 3TAA, the method comprising the steps of:
incubating a cell with the producer cell culture media described above with and without 3TAA;
determining the product titer of the cell in each media; and
comparing the product titer of the cell in each media.

Preferably, the effective amount of 3TAA in the producer cell culture media is between about 0.1 mM and about 6.0 mM.

Preferably, the effective amount of 3TAA in the producer cell culture media is between about 1.5 mM and about 3.5 mM. At this concentration, the producer cell culture media is generally added to the cell culture media at Day 0. Ideally, the effective amount of 3TAA in the producer cell culture media is about 2.5 mM when the producer cell culture media is added to the cells at Day 0.

Preferably, the effective amount of 3TAA in the producer cell culture media is between about 4.0 mM and about 6.0 mM when the 3TAA is added to cells at the mid to late exponential phase or early stationary phase of the cell growth following growth of the cells from Day 0 in a cell culture media lacking 3TAA. Ideally, the effective amount of 3TAA in the cell producer culture media is about 5.6 mM when 3TAA is added to cells at the mid to late exponential phase or early stationary phase of the cell growth. This can be Day 3 post-seeding or later, depending on the volume of the article (flask, well, plate, *etc.*) in which the cells are being incubated.

Preferably, the basal cell culture media is selected from Dulbecco's Modified Eagle Medium, Ham's F-10, Ham's F-12, Minimum Essential Medium, RPMI 1640 Media, CD CHO Media, FreeStyle™ 293 Expression Medium, CD FortiCHO medium, CD OptiCHO Medium, EX-CELL®, HyClone, Cellvento, BalanCD™ CHO Growth A, PowerCHO, ClonaCell, IS CHO-CD XP, CHOMACS CD. The producer cell culture media is one of the basal cell culture media selected from the above and having an effective concentration of 3TAA added to it.

Preferably, the cell culture media is protein-free and serum-free.

In one aspect, there is provided a use of an effective amount of 3-Thiopheneacetic acid (3TAA) in a cell culture media to increase product titer in a batch production process.

In one aspect, there is provided a use of an effective amount of 3-Thiopheneacetic acid (3TAA) in a producer cell culture media to increase production titer in a fed batch process.

In one aspect, there is provided a kit for increasing product titer of a protein-producing cell in a cell culture system, the kit comprising the producer cell culture media as described above.

### Definitions

The term "analogue thereof" as used herein should be understood to mean a functional and/or structural analogue of 3TAA. A functional analogue is preferably a carboxylic acid molecule with a thiophene/thienyl group. A functional analogue of 3TAA should deliver at least a 1.5-fold increase in titer production versus control when used at its optimal concentration and at optimum time of deployment, with a cell growth reduction of no more than 30%, 31%, 32%, 33%. 34%. 35%. 36%, 37%, 38%, 39% or preferably no more than 40%.

The term "effective amount" should be understood to mean an amount of 3TAA (or analogue thereof) in a cell culture media that increases the product titer of cells incubated in said cell culture media compared with the titer production of cells incubated in the same cell culture media that does not contain 3TAA. The effective amount can be 0.1 mM, 0.2 mM, 0.3 mM. 0.4 mM, 0.5 mM, 0.6 mM, 0.7 mM, 0.8 mM, 0.9 mM, 1.0 mM, 1.1 mM, 1.2 mM, 1.3 mM, 1.4 mM, 1.5 mM, 1.6 mM, 1.7 mM, 1.8 mM, 1.9 mM, 2.0 mM, 2.1 mM, 2.2 mM, 2.3 mM, 2.4 mM, 2.5 mM, 2.6 mM, 2.7 mM, 2.8 mM, 2.9 mM, 3.0 mM, 3.1 mM, 3.2 mM, 3.3 mM, 3.4 mM, 3.5 mM, 3.6 mM, 3.7 mM, 3.8 mM, 3.9 mM, 4.0 mM, 4.1 mM, 4.2 mM, 4.3 mM, 4.4 mM, 4.5 mM, 4.6 mM, 4.7 mM, 4.8 mM, 4.9 mM, 5.0 mM, 5.1 mM,. 5.2 mM, 5.3 mM, 5.4 mM, 5.5 mM, 5.6 mM, 5.7 mM, 5.8 mM, 5.9 mM, 6.0 mM.

The term "microtiter plate" or "multiwell plate" as used herein should be understood to mean plates have a plurality of reaction wells, generally at least 12, 24, 48 or 96 wells, each well generally having have a volume of less than 5, 4, 3, 2 or 1ml. The term should be understood to include rigid plates, and plates provided in the form of a flexible roll. The plates can be incubated in static or shaking conditions.

In this specification, the term "cell" refers to any cell type, including prokaryotic or eukaryotic cells. Suitably, the cell is a eukaryotic cell, ideally a mammalian cell. Typically, the cell is a producer cell, preferably a mammalian producer cell. The cell may be clonally-derived or non-clonal. In a preferred embodiment, the cell is a clone from a panel of clonal cells, derived from a single parental cell population or derived from different transfected cells. The cell may also be from a distinct cell line, for example a genetically modified cell (*i.e.* cells with "knock-out" or "knock-in" mutations). The cell line may also be derived by directed evolution, by selection of cells which have adapted to a specific environment of interest. The term "panel of distinct cells" should be understood to mean a panel of cells that are different from each other. For example, the panel of cell may comprise a panel of clones that are all derived from a single parental cell population. Alternatively, the panel of cells may comprise cells of independent clonal origin, for example cells carrying different transgenes or different mutations, of the panel of cells may comprise non-clonal cells. The cell line may also be derived by directed evolution, by selection of cells which have adapted to a specific environment of interest. The panel of cells may also comprise cells of different cell type, for example different cell lines, different strains of bacteria or fungi or plants, cells of the same cell type but at a different stage of development, and cells of the same cell type that differ genetically, for example cells of the same type or origin or cells derived from the same parental cell population that carry different transgenes.

The term "product titer" or "product titer response" as applied to a specific producer cell clone refers to the amount of a specific protein, generally a recombinant protein, and ideally a recombinant monoclonal antibody, that the specific producer cell clone generates over a defined time period. The titer may be quantified in absolute or relative terms. Generally, titer is referred to as weight of product per volume of culture - grams per litre (g/L) is a common metric (Max titer). Generally, the clones are incubated for a specific time period, for example 2-4 days, and following the incubation period a sample of the supernatant is typically taken and assayed for product titer. Various methods will be apparent to the person skilled in the art for measuring product titer, including HPLC and quantitative ELISA.

The term "producer cell" refers to a cell that is employed to generate a specific desired protein. Generally, the cell is genetically modified to include one or multiple copies of a transgene encoding the desired protein which is generally under the control of a specific promotor. Thus, the specific protein is usually a recombinant protein expressed stably by producer cells or expressed transiently following transfection of cells with gene of interest for protein of interest. Producer cells are well known in the art, and include, for example, mammalian cell hosts such as Chinese Hamster Ovary (CHO) cells, baby hamster kidney (BHK) cells, mouse myeloma cells (NS0, Sp2/0-Ag14), human lines like HEK 293 and PER-C6, yeast species *S. cerevisiae* and *Pichia pastoris;* and insect cell lines (for example, lepidopteron cell lines with a baculovirus to form the insect cell-baculovirus expression vector system (IC-BEVS) Drugmand JC, Schneider YJ, Agathos SN (2012) Insect cells as factories for biomanufacturing. Biotechnol Adv 30:1140-1157. doi: 10.1016/j.biotechadv.2011.09.014); or stable lines generated from Schneider 2 or Sf-9 cells and plant producer cells (for example, carrot plant root cell line (Elelyso, Pfizer); Walsh G (2014) Biopharmaceutical benchmarks 2014. doi: 10.1038/nbt.3040) and prokaryotic cell hosts like *E coli.* Ideally, the producer cell is a CHO cell. Typically, the producer cell is a monoclonal antibody producer cell.

The term "product titer response profile" refers to the product titer responses for a given clone to the addition of 3TAA (or an analogue thereof). In one embodiment, the profile may include a single production titer response for a given clone (the product titer response for the cell in the presence and typically absence of 3TAA (or an analogue thereof)). Generally, the profile is generated using normalized product titer responses (product titer in presence of 3TAA (or an analogue thereof) divided by product titer in absence of 3TAA (or an analogue thereof)), so that a normalized product titer response profile is obtained.

The method of the invention employs rapid profiling of producer cells to predict the product titer of the cells when in fed batch culture. In this specification, the term "fed batch" or "fed batch culture" should be understood to mean extended culture of cells where additional nutrients are added in bolus format at least once post seeding of the cells.

The term "calibration set of clones" refers to a plurality of clones, each having a product titer response fingerprint and a known fed batch performance. Typically, the calibration set of clones includes clones exhibiting a range of fed batch performance abilities from what the end user would consider 'good' performers to what the end user would consider 'bad' performers. Suitably, the calibration set of clones comprises at least 3, 4, 5, 6, 7, 8, 9, 10, 12, 14, 16, 18, 20, 22, 24, or 26 clones.

In this specification, the term "rapid" should be understood to mean a method of predicting fed batch performance that takes less than ten days, and ideally five days or less post-addition of the producer cell culture media of the invention.

In this specification, the term "high-throughput" should be understood to mean a method in which a large number of samples, for example 20-500, can be assayed simultaneously. In one embodiment, this involves assaying the growth of the clones in a multiwell plate, for example a 48, 96, or 192 well plate.

In this specification, the term "predicted fed batch product titer" as applied to a clonal producer cell should be understood to mean the predicted fed batch product titer of the clone in fed batch culture. The predicted product titer may be quantified in absolute terms, or may be quantified in relative terms, *i.e.* relative to a clone having known good or bad product titer or relative to a panel of clones.

In this specification, the term "predicting relative product titer" should be understood to mean predicting fed batch product titer of the clones in the panel relative to one another. Thus, the clones can be stratified according to their predicted fed batch product titer. Likewise, the term "predicted relative fed batch product titer of a panel of clonal cells" should be understood to mean the predicted fed batch product titer of the clones in the panel relative to one another. Thus, the clones can be stratified according to their predicted fed batch product titer. In another embodiment, the clones are stratified to pick one or more clones showing best predicted fed batch product titer clones, one or more clones showing worst predicted fed batch product titer clones, or both.

In this specification, the term "panel of clonal producer cells" should be understood to mean a panel of clonal producer cell populations derived from a single cell line and comprising from 2 to 500 or more clonal producer cell populations. Methods for generating panels of clonal producer cells are well known to a person skilled in the art and described in Production of recombinant protein therapeutics in cultivated mammalian cells (2004), Wurm, Florian M, New York, NY, Nature Biotechnology 22 (2004), S. 1393-1398. Typically, the panel of clonal producer cell populations include from 10-500, 20-500, 30-500, 40-500, 50-500, 60-500, 70-500, 80-500, 90-500 or 100-500 clonal cell populations. Typically, the panel of clonal producer cell populations include from 100-500, 100-400, 150-400, 150-350 clonal cell populations.

In the specification, the term "Day 0" should be understood to mean the day on which the cells are seeded in a culture flask or deep well plate for culturing.

In the specification, the term "Day 3", "Day 4", "Day 5", "Day 6" *etc.* should be understood to mean the third, fourth, fifth, sixth, *etc.* day post-seeding of cells in culture.

In the specification, the term "cell culture media" or "basal cell culture media" should be understood to mean a media designed to support the growth of producer cells (microorganisms or mammalian cells or plant cells or insect cells). Typically, the cell culture media contains a carbon source (such as glucose, glutamine or succinate), water, a nitrogen source (such as amino acids), vitamins, various salts (for example, an isotonic solution of saline), and water. Phenol Red dye may be added as a pH indicator, and a bicarbonate/HEPES buffer may be added to maintain a balanced pH. Examples of suitable cell culture media for producer cells include Dulbecco's Modified Eagle Medium, Ham's F-10, Ham's F-12, Minimum Essential Medium, RPMI 1640 Media, CD CHO Media, FreeStyle™ 293 Expression Medium, CD FortiCHO medium, CD OptiCHO Medium, EX-CELL®, HyClone, Cellvento, BalanCD™ CHO Growth A, PowerCHO, ClonaCell, IS CHO-CD XP, CHOMACS CD.

In the specification, the term "period of time" should be understood to mean a period of incubation of 10 days or less , that is, a period of incubation of between 1, 2, 3, 4, 5, 6, 7, 8, 9 or 10 days. Ideally, the period of time is the period of incubation following the addition of the cell culture media, containing 3TAA, which coincides to when the cells are in mid to late exponential phase or early stationary phase of the cell growth. For example, if incubating the cells in plates, the period of time for post-feeding of cells would be at Day 3. In flasks, the batch growth profile is different so the ideal period of time for post-feeding could be at Day 5, while in fed batch cultures it could be Day 6. Ideally, the producer cells are ideally fed with the producer cell culture media of the invention when the cells are at mid or late exponential phase or early stationary phase of the cell growth.

### Brief Description of the Drawings

The invention will be more clearly understood from the following description of an embodiment thereof, given by way of example only, with reference to the accompanying drawings, in which:
**Figure 1** illustrates the effects of different concentrations of 3TAA on culture performance in deep well shaking plate culture. Chemical enhancer was added on Day 0. Mean ± standard error of two experimental replicates each with three technical replicates.
**Figure 2** illustrates delayed addition of chemical enhancer molecules. Effect on culture attributes in the presence of 3TAA when added day 3 post seeding. Mean ± standard error of three experimental replicates each performed in triplicate.
**Figure 3** illustrates culture performance of 3TAA supplemented cultures in batch production process. (A) Viable cell density over culture progression (B) Cell viability for the culture duration. (C) Integral of viable cell density (IVCD) - a measure of accumulation of viable cell biomass as the culture progresses. (D) Volumetric IgG titer.
**Figure 4** illustrates the efficacy of 3TAA in 3TAA-supplemented cultures in a batch production process in comparison to similar molecules. Parallel studies were performed with 2TAA (a structural analogue of 3TAA) to compare against culture performance of 3TAA. (A) Viable cell density over culture progression (B) Cell viability for the culture duration. (C) Integral of viable cell density - a measure of accumulation of viable cell biomass as the culture progresses.
**Figure 5** illustrates cell cycle phase profile of cells in the presence of enhancer chemical. Flow cytometric analysis revealed which phase of cell cycle was predominant in each cell. Mean ± standard error of three experimental replicates each performed in duplicate.

### Detailed Description of the Drawings

### Materials and Methods

### Determining of optimum concentration in culture

The Applicant uses a platform consisting of screening Chinese Hamster Ovary (CHO) cell clone leads grown in a deep well plate coated with media containing 3TAA. The cells are measured for increased growth and productivity (titer) to determine whether 3TAA, and other enhancer chemicals, have offered the optimum cell culture environment, and can improve the growth and/or productivity of promising lead clones, which otherwise, grown in sub-optimal conditions, would not have been selected. To establish optimum concentration for the best titer boost, high throughput experimentation was performed in 96 deep well plates (Greiner Bio-One, Gloucestershire, United Kingdom). The cell line used was a CHO-S stable clone producing an anti-Her2 like IgG1 antibody. Cells were cultured in CD CHO medium (Thermo Fisher Scientific, Paisley, UK), supplemented with 8 mM L-glutamine, 1% HT supplement and 12.5 ug/ml puromycin selection marker (Thermo Fisher Scientific, Paisley, UK). Cells grown in these plates were incubated at 320 rpm (25mm throw), 37°C, 5% CO₂ and 85% humidity. The plates were covered with vented lids and secured using clamps ("System Duetz"; Enzyscreen B.V., Heemstede, Netherlands). Cells were seeded at 0.2×10⁶ cells ml⁻¹ with a seeding volume of 450 µL (unless specified otherwise). Chemical enhancer molecules, including 3TAA, were trialled at 4 to 10 different concentrations at Day 0 in culture. Subsequently, a subset of concentrations was carried forward for delayed addition at Day 3 of culture. Day 3 was chosen based on the cell growth profile in deep well plates. Cells were cultured for 5 days before culture attributes (cell growth and titer) were recorded, unless otherwise stated. Cell growth was measured using the PrestoBlue® Assay (ThermoFisher Scientific), viability was verified using the Vi-cell™ cell viability analyser (Beckmann Coulter, High Wycombe, UK), and titer was measured using the Valita™TITER assay (Valitacell, Dublin, Ireland).

### Analysis of 3TAA supplemented cultures in shake flasks

Shake flask cultures were performed in vented Erlenmeyer flasks with a 30 ml working culture volume. Cells were seeded at 0.2×10⁶ cells ml⁻¹. 3TAA was added at 2.5mM (optimum concentrations from deep well plate culture) on Day 0. Growth and titer were monitored daily. Total RNA and histone extracts were collected from Day 5 of culture. Cell cycle and apoptosis analysis were performed on Day 4 and Day 5 post seeding respectively.

### RNA extraction, cDNA synthesis and qPCR

Cellular total RNA was extracted from 1×10⁶ cells using the RNeasy® Mini Kit (Qiagen, Crawley, UK) according to the manufacturer's instructions. Reverse transcription reactions were carried out using the QuantiTect® Reverse Transcription kit (Qiagen) according to the manufacturer's instructions. Real time quantitative PCR (qPCR) reactions were carried out using the QuantiFast® SYBR® Green PCR Master Mix according to the manufacturer's instructions. Primers for cDNA corresponding to the recombinant antibody heavy (Fwd: ACCAAGAACCAGGTCAGCCT (SEQ ID NO. 1), Rev: TGAGAAGACGTTCCCCTGCT (SEQ ID NO. 2)) and light chain (Fwd: CAGCAAGGACAGCACCTACA (SEQ ID NO. 3); Rev: GACTTCGCAGGCGTAGACTT (SEQ ID NO. 4)) mRNA were employed. Internal reference controls used were: Mmadhc (Fwd: TGTCACCTCAATGGGACTGC (SEQ ID NO. 5); Rev: CAGGTGCATCACTACTCTGAAAC (SEQ ID NO. 6)) and Fkbpla (Fwd: CTCTCGGGACAGAAACAAGC (SEQ ID NO. 7); Rev: GACCTACACTCATCTGGGCTAC (SEQ ID NO. 8)). For the internal reference primer information, see reference Biotechnol J. 2018 Jan;13(1). doi: 10.1002/biot.201700259. Transcriptome-Based Identification of the Optimal Reference CHO Genes for Normalisation of qPCR Data.

### Cell cycle analysis

1×10⁶ cells were extracted from culture and fixed in 4% paraformaldehyde (Alfa Aesar; Thermo Fisher Scientific). The cells were then stained with propidium iodide using the FxCycle™ PI/RNase Staining Solution (Thermo Fisher Scientific). Cell cycle analysis was performed on the Attune Acoustic Focusing Cytometer (Thermo Fisher Scientific).

### Results

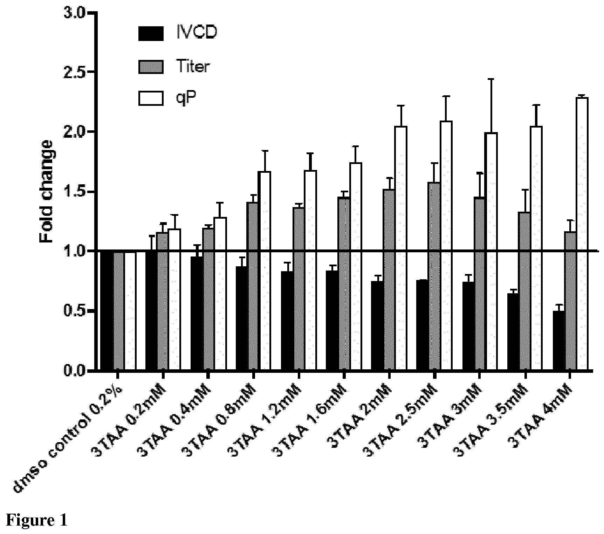

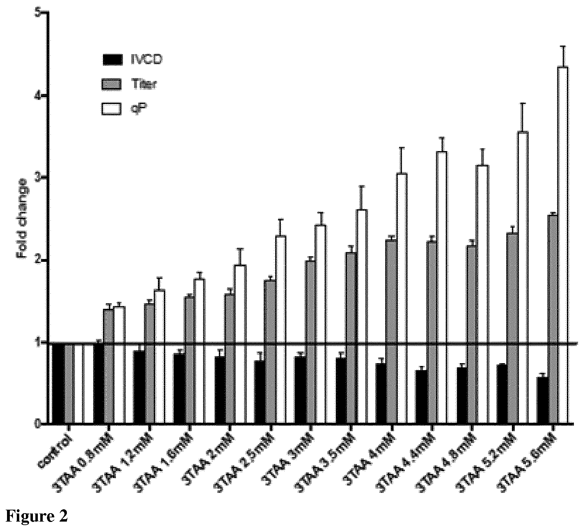

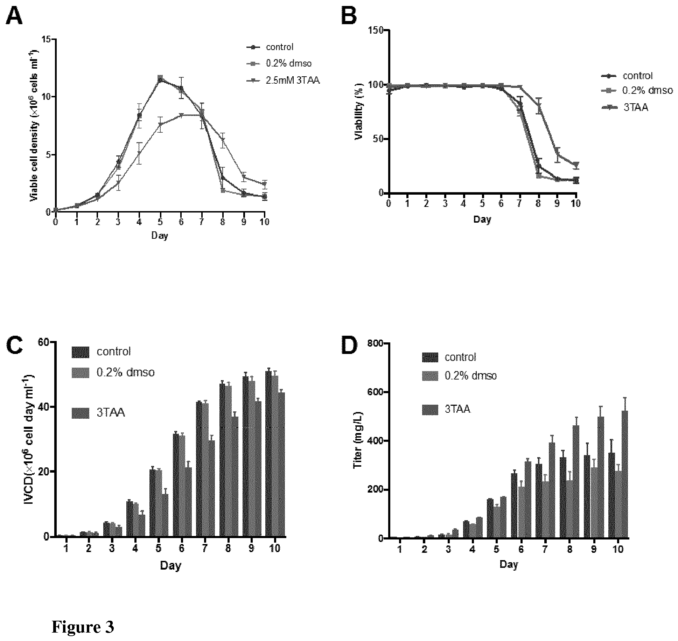

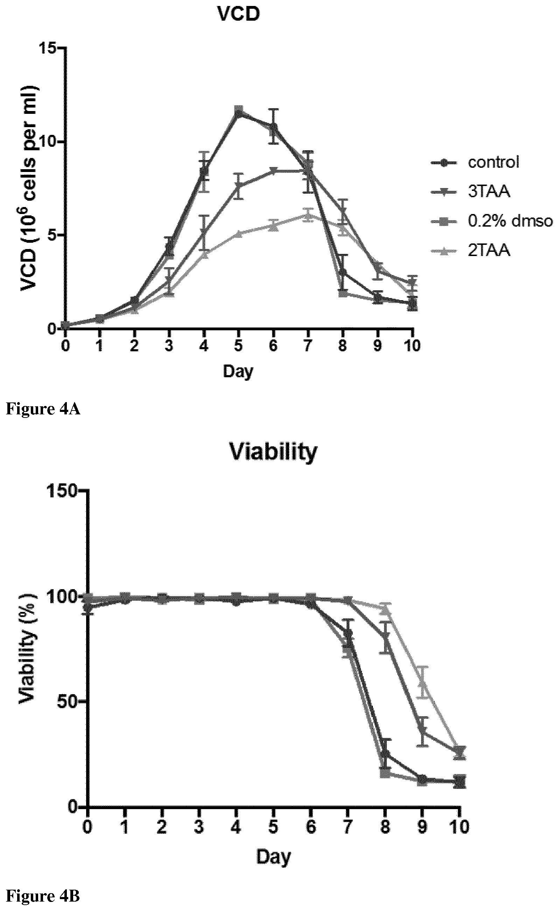

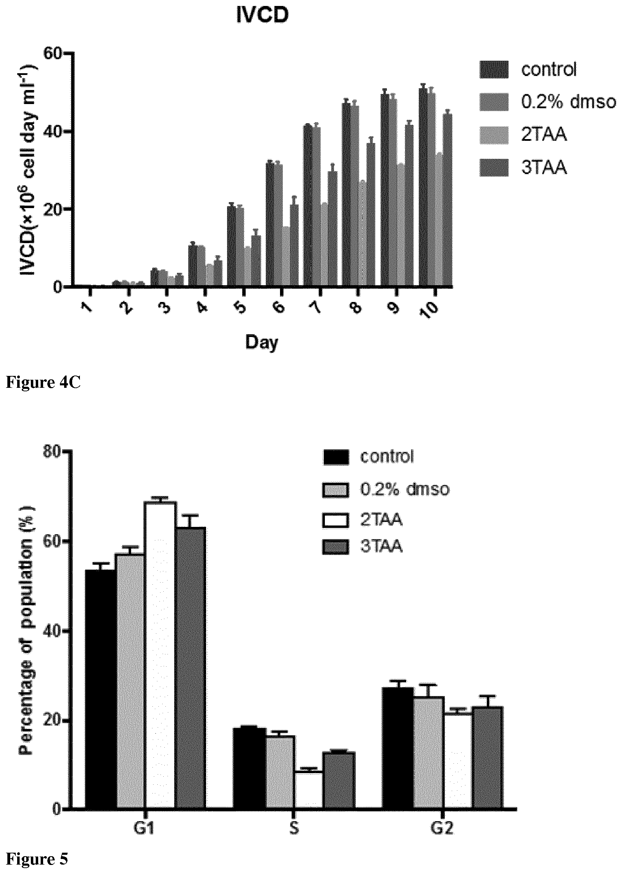

**Example 1:** High throughput culture was performed to determine the optimum concentration (Day 0 addition) of 3TAA. 3TAA was trialled in a deep well shaking culture setup to determine the best concentration for titer boost. 3TAA (Figure 1) when added at the start of culture yielded the best titer boost of 1.59-fold (2.5mM). No detrimental effect was observed in viability up to 5.2mM. Cell growth was reduced by 25%.
**Example 2:** Delayed addition of 3TAA was performed using an IgG producing stable cell line, adding the chemical molecule at the exponential phase of culture progression (see Figure 2). This approach revealed a larger improvement in volumetric titer, with 3TAA addition producing a 2.6-fold titer boost. Cell growth was dampened to a lesser extent in comparison to the Day 0 addition cultures. For example, at 2.5mM concentration, Day 0 addition reduced IVCD by 25% with a titer boost of 160%. Conversely, a Day 3 addition at the same concentration yielded at 20% reduction in IVCD, boosting titer by 177%. Further, at the 4mM concentration, Day 0 addition reduced IVCD by 50% with a titer boost of 17%. Conversely a Day 3 addition of the same concentration yielded a 25% reduction in IVCD, boosting titer by 125%.
**Example 3:** In a batch culture study (see Figure 3), the selected optimum concentration of the 3TAA molecule was used in a batch shake flask process lasting 10 days. The molecule was added on Day 0. 3TAA decreased peak viable cell density by 30%, increasing total volumetric titer by 1.9-fold. Culture period was prolonged by a day due to the slower growing cells. The culture viability remained unaffected in comparison to the vehicle and no addition controls; demonstrating that prolonged exposure to the chemical did not deteriorate cell viability.
**Example 4:** To determine efficacy in comparison to similar molecules, parallel studies were performed with 2TAA (a structural analogue of 3TAA) supplemented cultures (see Figure 4). For Day 0 addition cultures, 2TAA recorded a titer increase of 1.52-fold (at 0.8mM), consistent with 3TAA titer increase (1.59-fold, at 2.5mM). Growth decreased by 49% with 2TAA, in comparison 3TAA recorded a 25% growth decrease. For 2TAA, concentrations of 1.6mM or higher recorded a significant drop in viability. In comparison, viability of cultures supplemented with 3TAA was unaffected until 5.2mM.
   In the case of exponential phase addition, 2TAA at 2mM produced a titer improvement of 3.2-fold over the vehicle control. However, it is important to note that at this optimal concentration, 2TAA hampered cell viability over time in culture making it an unsuitable media supplement for improving titer production.
**Example 5:** For cell cycle analysis, cellular samples were fixed, stained and analysed through flow cytometry (see Figure 5). Cell cycle phase distribution revealed that there was an 11% increase in accumulation in the G1 phase. Accumulation in the G1 phase forms a valid explanation for the decreased cell growth and increase in cell diameter. 3TAA can be employed for use in a cell culture production process. The inventors have shown that 3TAA has provides a significant improvement in volumetric titer. Apoptotic pathways were not initiated, making 3TAA extremely valuable as a small molecule enhancer. Host cell lines stably/transiently producing a biotherapeutic of interest, can be cultured in the presence of 3TAA for improvement in protein yields. 3TAA can function as a culture additive. It can also form part of a chemical feed to administer to cells at a later stage in culture, to allow cellular resources to solely focus on protein production after a proliferation phase.

As far as the inventors are aware, 3TAA has never been trialled as a media supplement in mammalian cell lines. This is the first instance of its reported ability to improve titer in mammalian cell hosts producing a therapeutic antibody.

In the specification the terms "comprise, comprises, comprised and comprising" or any variation thereof and the terms "include, includes, included and including" or any variation thereof are considered to be totally interchangeable and they should all be afforded the widest possible interpretation and vice versa.

The invention is not limited to the embodiments hereinbefore described but may be varied in both construction and detail.

## Claims

1. A producer cell culture media comprising an effective amount of 3-Thiopheneacetic acid (3TAA).

2. The producer cell culture media according to Claim 1, in which the effective amount of 3TAA is between about 0.1 mM and about 6.0 mM.

3. The producer cell culture media according to Claim 1 or Clam 2, in which the effective amount of 3TAA is between about 1.5 mM and about 3.5 mM when the producer cell culture media is added to cells at Day 0.

4. The producer cell culture media according to Claim 3, in which the effective amount of 3TAA is about 2.5 mM when the producer cell culture media added to the cells at Day 0.

5. The producer cell culture media according to Claim 1 or Claim 2, in which the effective amount of 3TAA is between about 4.0 mM and about 6.0 mM when the producer cell culture media is added to the cells at mid to late exponential phase or early stationary phase of the cell growth.

6. The producer cell culture media according to Claim 5, in which the effective amount of 3TAA is about 5.6 mM.

7. The producer cell culture media according to any one of the preceding claims, in which the producer cell culture media is in the form of a powder, a liquid, a pellet, a granule.

8. A method for increasing product titer in a cell culture system, the method comprising the steps of:
(a) incubating a cell in the producer cell culture media as claimed in Claim 1 for a period of time in the cell culture system;
(b) collecting the cell or cell culture media or both from the cell culture system;
and (c) harvesting the product produced by the cell in the cell culture system.

9. A method for increasing protein production of a protein-producing cell in a cell culture system, the method comprising the steps of:
(a) incubating the protein-producing cell in the producer cell culture media as claimed in Claim 1 for a period of time;
(b) collecting the producer cell or producer cell culture media, or both, from the system; and
(c) harvesting the protein from the producer cell or producer cell culture media, or both.

10. The method of Claim 9, in which the harvested protein is a monoclonal antibody, a polyclonal antibody or a recombinant protein.

11. The method of any one of Claims 8 to 10, in which prior to step (a), the cells are first incubated with a basal cell culture media without 3TAA for a period of time until the cells are at mid to late exponential phase or early stationary phase of the cell growth and then step (a) is performed.

12. A method of determining whether the producer cell culture media of Claim 1 increases product titer in a cell culture system when compared to product titer when using the producer cell culture media without 3TAA, the method comprising the steps of:
incubating a cell with the producer cell culture media of Claim 1 with and
without 3TAA;
determining the product titer of the cell in each media; and
comparing the product titer of the cell in each media.

13. The method according to any one of Claims 8 to Claim 12, in which the effective amount of 3TAA in the producer cell culture media is between about 0.1 mM and about 6.0 mM.

14. A use of an effective amount of 3-Thiopheneacetic acid (3TAA) in a producer cell culture media to increase production titer in a batch production process or in a fed batch process.

15. A kit for increasing production titer of a protein-producing cell in a cell culture system, the kit comprising the producer cell culture media as claimed in any one of Claims 1 to 7.
